# EUROPEAN PATENT APPLICATION

(11) **EP 1 563 851 A1**
(43) Date of publication of application: **17.08.2005**
(21) Application number: 04290427.6
(22) Date of filing: 17.02.2004
(51) Int. Cl.: A61K 47/48

(54) **Antibody linked cationic emulsions as drug delivery system**

(71) Applicant: Yissum Research Development Company of the Hebrew University of Jerusalem, 91042 Jerusalem (IL); Novagali Pharma SA, 91000 Evry (FR)
(72) Inventor: Benita, Simon, 3 Mevasseret Sion 90805 (IL); Goldstein, Danny, Rehavia, Jerusalem 94188 (IL); Nassar, Taher, P.O. Box 464 464 (IL); Sader, Ola, 2 Haifa 35702 (IL); Razafindrastsita, Alain, 94550 Chevilly La Rue (FR); Lambert, Gregory, 91370 Verrieres Le Buisson (FR); Kadouche, Jean, 75013 Paris (FR)
(74) Representative: Warcoin, Jacques

(57) **Abstract**

A combination product comprising a positive oil in water emulsion wherein said emulsion comprises a compound presenting free NH₂ groups, at its natural state, at the oil-water interface, and an antibody, wherein said compound is linked to said antibody by a heterobifunctional linker, linking said NH₂ groups to SH groups on the antibody hinge region.

## Description

This invention relates to both a drug delivery system and a method for manufacturing such a drug targeting system.

The invention also relates to a useful method of targeting and delivering drugs, diagnostics and other physiologically effective substances to a target within the mammalian body.

Cancer is one of the leading causes of death in the world and the second one in the United States. Among the different cancer diseases, breast cancer is the most common cancer in women except for melanoma skin cancer. Pancreatic cancer is the forth leading cause of cancer death in the US and colorectal cancer is expected to cause 57 000 deaths during 2003. The conventional cancer therapies are aggressive, induced significant side effects and even cause damage to healthy tissues at the vicinity of the malignant organs to be treated. The demand for increased specificity of anticancer agents to target tumors has resulted in numerous innovative therapeutic strategies. Recently a new and exciting biotechnology era is emerging with the approval of monoclonal antibodies (MAb) for therapeutic applications especially in cancer [1]. It has been observed over the last two decades that progression of cancer is often accompanied by the over-expression of one or several proteins, called tumor antigens [2]. A leading MAb directed against the p185^{HER2} (HER2) receptor tyrosine kinase, trastuzumab has been developed. HER-2 plays an important role in the pathogenesis of breast, pulmonary, ovary and other cancers [3] and is over-expressed in such cancers. HER-2 over-expression is clearly associated with poor prognosis in breast cancer [4, 5]. Therefore, the use of monoclonal antibodies for the treatment of cancer has been suggested as a means of targeting cancer cells while sparing normal cells. Among the MAbs in the market, rituximab and trastuzumab (Rituxan® and Herceptin®, respectively) exhibit promising and encouraging results in the treatment of cancer and continue to expand significantly [6].

In some cases, the use of MAb as single agent is not sufficient to produce a satisfactory therapeutic response. Furthermore, in preclinical studies with tumor cell lines, trastuzumab, was found to have additive and synergistic effects with some chemotherapeutic agents [2,7]. Clinical trials investigating combination of trastuzumab with a variety of chemotherapeutic agents especially, paclitaxel, are already in progress in lung cancer [8]. Investigators have taken advantage of the MAbs high affinity for tumoral tissues and designed innovative and efficient therapeutic strategies by coupling MAb to toxins or to a radioactive isotope that destroys antigen carrier cells by irradiation (radio immunotherapy) [9]. Numerous clinical studies have recently reported promising results on the therapeutic use of yttrium-labeled polyclonal antiferritin in patient with recurrent Hodgkin's disease (HD) [10] despite conflicting data regarding the over-expression of H-ferritin in malignant cells [11]. Thus, in an attempt to achieve more efficient tumor drug targeting, monoclonal antibodies are being coupled with colloidal carriers such as liposomes (immunoliposomes), nanoparticles and emulsions (immunoemulsions) able to entrap potent hydrophilic and /or lipophilic drugs. These immunoconjugates should ensure the specific recognition of the antigen site by the antibody and the release of different cytotoxic agents by the colloidal delivery system close to the inaccessible pathological target tissues, which may be located in the breast, colon or pancreas over-expressing such tumor antigen. Indeed, immunoliposomes represent a novel and promising strategy for enhanced tumor targeting drug delivery as recently reported [12-19]. Furthermore, it has been showed that immunoliposomes bearing polyethyleneglycol-coupled Fab' fragments show prolonged circulation time and high extravasation into targeted solid tumors in vivo [15]. However, the well-known physicochemical instability of these sensitive liposomal preparations remain an obstacle which needs to be overcome if viable products are intended to be ultimately marketed. In addition, most of these liposomal carriers cannot incorporate significant doses of lipophilic/hydrophobic active ingredients such as paclitaxel, one of the most promising cytotoxic drugs for the treatment of solid tumors in various cancers, limiting their potential clinical efficacy while emulsions can incorporate significant levels of hydrophobic drugs especially paclitaxel [20] emphasizing their advantages over the liposomes. Lundberg et al. [21] coupled a negatively charged long-circulating submicron emulsion stabilized with polyethylene glycol modified phosphatidylethanolamine with an anti-B-cell lymphoma MAb LL2. They showed that the conjugate might be a useful drug-carrier system for more specific delivery of anti-cancer drugs to B-cell malignancy. No *in vivo* data have yet been presented and the study is still in the preliminary phase.

Cationic submicron emulsions developed and investigated by the inventors are stable in the presence of physiological cations and can interact in vivo with negatively charged biological membranes, while escaping from RES uptake [22]. In addition, it was shown in cell culture studies that the cationic emulsion enhanced the intracellular penetration of therapeutic agents [23, 24].

The HER-2 protein represents an attractive target for immunologically based antitumor therapy owing to its limited expression in non malignant tissues and its contribution to the malignant phenotype of a transformed cell. Trastuzumab which targets HER-2/neu in combination with chemotherapy confers a survival benefit for patients with metastatic breast carcinoma that over-expresses HER-2, raising the possibility of therapeutic benefits for other type of malignancies that similarly may express HER-2. Furthermore, in other cancers, it was shown that trastuzumab is not effective as a single agent.

AMB8LK antibody shows high affinity towards H-Ferritin. Ferritin is a protein expressed by most human cells and which serves as intracellular iron storage. Structural data of the molecule show that it is a macromolecule composed of a glycoproteinaceous shell, called apoferritin, which contains a metallic iron atom nucleus. Apoferritin has a MW of 440 kD. There are two types of subunits, called H and L, assembled in variable proportions. Ferritin is either acidic or basic according to the subunit type. While the role of basic ferritin is well known, the exact role of acidic ferritin is being elucidated only recently. Early views of the relationship between ferritin and cancer stem from work demonstrating an increase in total ferritin and well as a shift toward acidic (H-rich) ferritin in the serum of patients with various malignancies [26]. Subsequent evaluations of ferritin levels in tumor tissue itself have revealed a complex, perhaps disease-specific picture: for example, in some cases such as colon cancer [27], testicular seminoma [28] and breast cancer [29-30], increases in ferritin in tumor tissue versus comparable normal tissue have been reported. Vriensendorp and Quadri recently reported [10] that in Hodgkin's disease, circulating ferritin levels are in the range of 500 ng/ml. Stochastic considerations indicate that iv administration of 2.5 mg rabbit antihuman ferrtin IgG forms mainly one to one immune complexes in the circulation [31]. The antigen-antibody complex does not interfere with tumor targeting or lead to immune complex disease. Furthermore, Vriesendorp and Coll. [32] have shown in a clinical study that radiolabeled antiferritin targets tumor interstitium and shrink tumor by radiation and not by immunologic effects in recurrent HD patients. Less than 5 % of injected radiolabeled antiferritin is eliminated in the urine of patients with HD. In addition, the 40 h effective secondary half-life of blood radioactivity and an α:β ratio below 2.5 indicate that radiolabeled rabbit antiferritin is stable *in vivo* [33,34]. Finally, antiferritin also targets the ferritin found in normal testes and blood but it has been observed in HD patients that blood pool radioactivity correlates well with hematological toxicity which usually disappears spontaneously by 10-16 weeks. No acute side effects were noted in the HD patients [10, 31]. It should be stressed that the antibody used in the previous clinical studies was a polyclonal rabbit antihuman ferritin. AMB8LK, a monoclonal antibody produced by Monoclonal Antibodies Therapeutics (MAT) Ltd. Evry, France disclosed in WO 01/52889, recognizes a common epitope to all the isoferritins (acidic and basic). AMB8LK has been shown to exhibit marked affinity for specific organs and following coupling with isotopes, it can be used in the diagnosis and treatment of Hodgkin's disease and other tumors like pancreas and lung (NSCLC) cancers.

Finally, the Orphan Drug Status has been recently granted by the EC Orphan Medicinal Products Committee for coupling the anti-ferritin polyclonal antibody to yttrium 90 for the treatment of refractory Hodgkin's disease (MAT, ltd., Evry France). This recognition provides convincing evidence that anti-ferritin can be preferentially uptake by tumors of HD patients.

There is a need to selectively target anticancer drug loaded emulsions via specific ligands against antigens expressed on malignant cells to improve the therapeutic effectiveness of the immunoemulsion preparations as well as reduce adverse side effects associated with chemotherapy.

It is therefore an object of the present invention to provide compositions and methods of targeting drugs.

The present invention relates to a combination product comprising a positive oil in water emulsion, wherein said emulsion comprises a compound presenting free NH₂ at its natural state, at the oil-water interface, and an antibody, wherein said compound is linked to said antibody by a heterobifunctional linker, linking said NH₂ groups to SH groups on the antibody hinge regions.

The invention more specifically relates to a combination product wherein said product has a positive zeta charge.

The invention also relates to a combination product, wherein said compound presenting NH₂ free groups is at least one cationic lipid selected from the group consisting of a C₁₀-C₂₄ alkylamine, a C₁₀-C₂₄ alkanolamine and a cholesterol ester.

The invention also relates to a combination product wherein said compound presenting NH₂ free groups is stearylamine or oleylamine.

In addition, the invention relates to a combination product wherein said emulsion comprises colloid particles having an oily core surrounded by an interfacial film, wherein said interfacial film comprises said compound presenting free NH₂ groups at its natural state, non ionic surfactant and an anionic surfactant or anionic lipid, wherein said colloidal particles have a positive zeta potential.

Such emulsions are disclosed in the international application WO 03/053405.

More particularly, the present invention teaches a combination product wherein said emulsion contains a pharmacologically active substance.

The present invention also relates to a combination product wherein said antibody is selected from the group consisting of polyclonal antibody and monoclonal antibody. The monoclonal antibody may be used under a native form, a synthetic form, a chimeric form or a humanized form.

More particularly, in the combination product according to the instant invention, the antibody targets an antigen present at the surface of a pathological cell.

More specifically, in the combination product according to the instant invention, said antibody targets a protein selected from the group comprising HER-2, H-ferritin, Prostate-specific membrane antigen (PSMA) or mucins (high molecular weight glycoproteins characterized by a high degree of glycosylation. MUC 1 is the best characterized of the nine mucins cloned so far. MUC 1 is over expressed in prostate cancers and in metastatic prostate cancers), CD 44 (a cell surface antigen expressed on Leukemia cells in all seven AML (Acute Myeloid Leukemia) subtypes) and retinal S-antigen (Monoclonal antibodies produced by hybridomas generated from retinal S-antigen (S-Ag) showed strong specific binding to the retinal Muller cells of all species tested (human, bovine, guinea pig and rat), [35].

More specifically, said antibody is the AMB8LK antibody, even more specifically the fragment form F(ab)2 obtained after digestion by pepsin.

The invention also relates to a combination product wherein said heterobifunctional linker is chosen in the group consisting of N-1 stearyl-maleimide (SM), oleylmaleimide, succinimidyl trans-4-(maleimidylmethyl)-cyclohexane-1-carboxylate (SMCC) and succinimidyl 3-(2-pyridyldithio)propionate (SPDP).

In addition, the invention relates to a method for producing combination products as disclosed before comprising the steps of:
a) optionally reducing an antibody in order to obtain free SH group on its hinge region,
b) mixing a positive emulsion wherein said emulsion comprises a compound which, at its natural state, contains free NH₂ groups, wherein said compound is linked to a heterobifunctional linker by said NH₂ groups, with the antibody presenting free SH groups in order to obtain said combination product.
Step a) is realised according to known techniques as for example the technique disclosed by Hermanson [36].

More particularly, the present invention teaches a method wherein said positive emulsion in step b) is obtained by:
i. linking a linker to a free NH₂ naturally present on a compound that is used to obtained a positive emulsion, in order to obtain a modified compound,
ii. mixing said modified compound, which at its natural state contains free NH₂ groups, with the other products necessary to obtain an emulsion, in order to obtain a positive emulsion.

The invention also relates to a method wherein said positive emulsion in step b) is obtained by:
i. mixing a compound, which at its natural state contains free NH₂ groups, with the other products necessary to obtain an emulsion, in order to obtain a positive emulsion,
ii. linking a linker to a free NH₂ group naturally present on said compound, in order to obtain a modified compound within said positive emulsion.

The reactive sulfhydryls in the hinge region are used in conjugation protocols with sulfhydryl-reactive cross-linking reagents bearing a maleimide group such as SMCC.

The rationale to synthesize the novel cross-linker reagents was to facilitate the formation of immunoemulsions and increase the yield of the reaction conjugation by mixing the new cross-linker very similar in chemical structure to the parent cationic lipid, i.e. stearylamine or oleylamine respectively during the emulsion preparation process. A mixed emulsifying interfacial film at the o/w interface is produced by the selected combination of four different surface active agents (phospholipids, poloxamer, stearylamine and strearyl maleimide or oleylamine and oleylmaleimide respectively).

In addition, one important step is saved in the conjugation reaction since there is no need any more to react a cross-linker with a preformed emulsion prior to the coupling of the antibody but to directly conjugate the reduced antibody to the emulsion comprising the cross linker already located at the o/w interface. It is then expected that no cross-reactions (between oil droplets and cross-linker or antibody molecules) will occur while the specificity and yield of the conjugation reaction are markedly increased since the reduced antibody faced directly the reactive moiety at the o/w interface of the emulsion.

According to the invention, the term pharmacological active substance comprises therapeutic agents and diagnostic agents, which may be employed with warm blooded animals, particularly mammal including humans, veterinarian animals or farm animals.

It is possible in accordance with the invention that the drug targeting system comprises one pharmacological active substance or more than one pharmacological active substance or even more pharmacological active substances, as long as they are compatible with each other in the same drug targeting system.

In a preferred embodiment of the drug targeting system of the invention, the pharmaceutically effective substances are lipophilic drugs selected from the group comprising gemcitabine, mitoxantrone, mitomycin, vincristine, epirubicin, methotrexate, etoposide, doxorubicin, purines or nucleosides, bleomycin, mitomycin, BCNU, taxol, taxotere and other taxane derivatives, more specifically paclitaxel oleate or palmitate, camptothecins, N4-acyl-1-beta-D-arabinofuranosylcystosines, dexamethasone, palmitate, triamcinolone (these two ones are for eye application), cyclosporine, tacrolimus, sirolimus (immunosuppressants with established P-gp inhibition effect, which reduce the multidrug resistance). All the drugs should be dissolved in the oil droplet core.

In an even more preferred embodiment, the pharmacological active substances are active cytotoxic substances that will be incorporated in these emulsions, prior to antibody conjugation, for example non aqueous soluble drugs such as paclitaxel, paclitaxel oleate and gemcitabine base alone or in combination with cyclosporine A or tacrolimus.

The advantages of coupling cationic emulsions containing either paclitaxel or gemcitabine with antibodies such as trastuzumab or AMB8LK that can specifically recognizes breast, colon and pancreas cancer tumors over-expressing either HER-2 or H-ferritin respectively or MUC 1 antibody or PMSA MAb able to recognize metastatic prostate cancer are multiple and include: immunotargeting by the transfer of the antibody specificity to the emulsion interface; prolonged residence time in the organism; release of large cytotoxic doses at tumor sites identified by the antibody and better cytotoxic drug internalization in tumors after biodegradation of the antibody from the emulsion interface and recovery of the drug-loaded positively charged emulsion which was shown to enhance drug penetration in various tissues.

The compositions according to the invention ensure, on the one hand, the specific recognition of the antigen site or the receptor by the antibody; and on the other hand, the release of different lipophilic cytotoxic agents by the colloidal delivery system close to the target pathological tissues. The drug release was triggered by the interaction of the immunoemulsion with the tumor cells. The therapeutic agent was anticipatedto be selectively transferred to the cell surface and subsequently internalized by constitutive endocytic or pinocytic invaginations of the plasma membrane, this ultimately delivering the cytotoxic agent to its site of action, which for most anticancer drugs is intracellular. The administration routes can be systemic routes (parenteral, intravenous, intraperitoneal, intra-rachidian, intra-tumoral), ocular or topical routes.

The efficient drug delivery system according to the invention significantly reduces side effects related to the non selective distribution of poorly tolerated and potent cytotoxic agents.

The advantages of coupling positively charged emulsions containing selected cytotoxic drugs with antibodies in oncology are multiple and include:
- immunotargeting by the transfer of the antibody specificity to the emulsion interface,
- prolonged residence time in the organism,
- release of large cytotoxic doses at tumor sites identified by the antibody,
- diminution of the emulsion positive charges and lung tropism,
- better cytotoxic drug internalization in tumors after biodegradation of the antibody from the emulsion interface and recovery of the drug-loaded positively charged emulsion that was shown to enhance drug penetration in various tissues.

The invention is further exemplified by the following examples and figures.

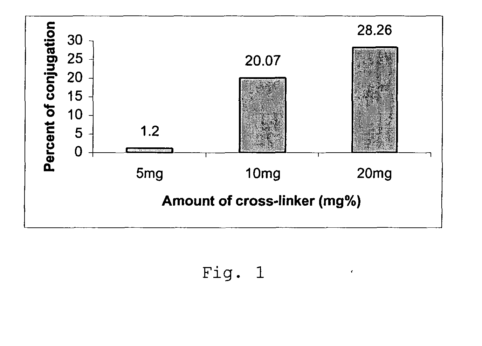

Figure 1 represents the percent of antibody molecules conjugation to oil droplets emulsion at an initial ratio of 45 trastuzumab molecules per oil droplet as a function of increasing cross linker SM concentration in the cationic emulsion.

Figure 2 is a schematic description of the coupling method of the positively charged submicron emulsion to a monoclonal antibody by the SMCC technique.

Figure 3 is a schematic description of coupling method of the positively charged submicron emulsion to a monoclonal antibody by the SPDP technique.

Figure 4 represents the uptake of different emulsion preparations by Capan-1-cells. The immunoemulsion has a density of 100 Ab/droplet.

### Example 1: Material and methods

### 1.1. Preparation of cationic emulsion.

The cationic blank emulsion is composed from an aqueous phase which includes distilled water, Poloxamer 188 (non ionic surfactant), glycerol (osmotic agent) and from an oil phase containing MCT (mid-chain triglycerides), stearylamine (cationic lipid), α-tocopherol (antioxidant) and Lipoid E-80 (mixture of phospholipids). The Lipoid E-80, Vit E and stearylamine are dissolved directly in the oil phase, whereas the poloxamer and glycerol are directly dissolved in the aqueous phase. Both phases were heated separately to 70°C. The water phase was slowly incorporated into the oil phase and mixed with a magnetic stirrer. The resulting mixture was further heated to a temperature of 85°C. The coarse emulsion obtained was emulsified over 5 minutes, using a high shear Polytron mixer (Kinematica, Luzern, Switzerland) and then rapidly cooled to below 20°C. After cooling in an ice bath, the emulsion was homogenized using a two-stage homogenizer valve assembly (Gaulin homogenizer, APV Gaulin, Hilversum, The Netherlands) at 9000 psi for 5 minutes. After further rapid cooling below 20°C, the pH was adjusted to 7.0 using 0.1N hydrochloric acid. The emulsion was then filtered through a TE membrane filter (Schleicher & Schuell, Dassel, Germany) with a pore size of 0.45 µm. Finally, the emulsion was packed under nitrogen atmosphere in siliconized glass bottles and sterilized by autoclaving at 121°C for 15 minutes.

The typical formulation consists of (w/w%): MCT (5), poloxamer 188 (1) glycerol (2.25), lipoid E80 (1), stearylamine (0.25), α-tocopherol (0.01) and water (up to 100).

For immunoemulsion preparation 0.02% of the synthesized cross-linker was added resulting in 4 000 cross-linkers per droplet.

For flow cytometry, fluorescence and confocal microscopy analysis, fluorescent labeled emulsion was prepared using coumarin 6 dissolved in tetrahydrofuran (THF) in a molar ratio of 1:200 with respect to the MCT content of the emulsion (final concentration of coumarin 490µM or 0.17mg/ml). The coumarin was allowed to equilibrate and penetrate within the emulsion over 2h of moderate heating while THF was removed by evaporation. Unloaded marker will be removed using Sephadex G-25 column.

### 1.2. Emulsion characterization.

### 1.2.1. Particle size analysis

Droplet size measurements were carried out utilizing an ALV Noninvasive Back Scattering High Performance Particle Sizer (ALV-NIBS HPPS, Langen, Germany) at 25°C and using water (refractive index: 1.332; viscosity: 0.894543) as the solvent. A laser beam at 632 nm wavelength was used. The sensitivity range was 0.5 nm to 5 µm.

### 1.2.2. Zeta potential measurements

The zeta potential of the emulsion was measured with the Malvern zetasizer (Malvern, UK) diluted in 10 mM NaCl (150 mV).

### 1.3. Preparation of anionic emulsions.

Anionic emulsions are prepared with oleic acid instead of Stearylamine using the same approach as described by Levy and Coll. [37]. The exact formulation is MCT 6.6%, oleic acid 2.3%, Lipoid E80 1%, Vit. E 0.01%, glycerol 2.25%, Pluronic F-68 1% and DDW 100%.

### 2. Preparation of antibody fragments.

Trastuzumab and AMB8LK F(ab)2 fragments reduction were performed according to the well-known method of Hermanson [36]. Purified trastuzumab and AMB8LK F(ab)2 antibody (4mg/ml) were reduced by 2-mercaptoethylamine (MEA, final concentration 0.05 M) for 90 min at 37°C to produce thiol groups for emulsion conjugation.

Once reduced with 2-mercaptoethylamine, immunoglobulins were cleaved in half, forming two heavy chain-light chain molecules of MW 75 000-80 000 and each containing one antigen binding site (it should be emphasized that this method does not lead to protein denaturalization). Similarly, F(ab')2 fragments may be reduced to yield two Fab' fragments, each containing an antigen binding site. The solution was eluted on a Sephadex G-25 column and Fab' fragments were collected in 1 ml fractions. Fractions containing Fab' were determined using a UV at 280 nm and pooled together. The Fab' fragments (50 kD) were kept under nitrogen atmosphere at 4°C until coupling to emulsion. Fab' fragments production was assessed by SDS-PAGE and their antigenic specifity was verified by sandwich ELISA.

### 3. Coupling reaction.

Freshly prepared emulsions according to 1 were adjusted to pH 6.5 with 1 N HCl and incubated with AMB8LK Fab' fragments (final concentration 0.1-0.5 mg/ml) overnight at 4°C under continuous agitation and under nitrogen atmosphere. Unreacted maleimide groups were blocked through incubation with 2-mercaptoethanol (2 mM) for 30 min. Unconjugated antibody and 2-mercaptoethanol were separated from immunoemulsions by gel filtration over a Sepharose CL-4B column. Morphological evaluation for the final immunoemulsion were done by means of confocal microscope and transmission electron microscopy (TEM) using FITC or gold labeled goat anti-mouse/human IgG respectively. The total amount of Fab' fragment conjugated to emulsions was evaluated by ELISA. For preparation of immunoemulsions with various amounts of conjugated antibody, the initial ratio of Fab' to maleimide-activated emulsions was varied.

### 4. Drug incorporation.

Selected lipophilic or hydrophobic cytotoxic drugs such as cyclosporine A were first dissolved in the oil phase of the emulsions prior to the conjugation with the antibody in order to prepare drug loaded immunoemulsions for effective treatment of tumor cells the access of which is difficult by conventional chemotherapy.

### 5. Stability study of the emulsion-antibody conjugate.

The stability of the coupled emulsion was studied *in vitro* by accelerated tests such as elevation of temperature, stirring and also using long term storage assessment.

The following properties were examined: droplet size distribution, Zeta potential, pH and drug content using HPLC [36].

### 6. In vitro drug release kinetic evaluation.

The *in vitro* drug release profile from the cationic emulsion was carried out using an ultrafiltration technique at low pressure as follows: 0.4 ml of the medicated emulsion (containing 1 mg of cyclosporine A) was directly placed in a Amicon 8 200 stirred vessel (Amicon, Danvers, MA, U.S.A) containing 100 ml of release medium (maintaining sink conditions). At given time intervals, the release medium was filtered through the YM-100 ultrafiltration membrane at low pressure (less then 7.25 psi) using nitrogen gas. An aliquot of 1 ml of the clear filtrate was assayed for paclitaxel content using HPLC [38]. Membrane adsorption and rejection must be accounted for in order to accurately measure aqueous concentrations of drug therefore validation was preformed prior to the use of the ultrafiltration technique.

### 7. Cell culture studies.

### Immunostaining for H-ferritin and HER-2 over-expression determination.

H-ferritin over-expression will be evaluated in different cancer cell lines (CAPAN-1 for pancreas cancer, Caco-2 for colon cancer and SK-BR-3 for breast cancer) and in non-cancer cells such as fibroblasts and smooth muscle cells.

Cells were trypsinized after reaching confluence and transferred (10⁵ cells per well) into 16 wells plate, which were covered with 18 mm cover slips. Cells were left to adhere to the cover slip for 24 hours, 37°C, and 5% CO₂. Medium was discarded and fixation done using fresh 4% paraformaldehyde for 10 min. Cells were washed with PBS and self-fluorescence was blocked with 50 mM NH₄Cl following blocking with 5% BSA. Cells were washed and incubated with a 1:50 dilution of AMB8LK overnight at 4°C. Cells were washed and incubated with a 1:50 dilution of FITC conjugated goat-anti mouse IgG for 1 hour at room temperature. Secondary antibody was washed five times with PBS following mounting and then cells were taken for observation using either fluorescence microscope or confocal microscope. The same procedure was preformed for HER-2 determination using trastuzumab and a FITC conjugated goat anti-human IgG as a secondary antibody.

### 8. Binding analysis of AMB8LK-immunoemulsions in vitro.

AMB8LK-immunoemulsion and control emulsions (cationic emulsion and anionic emulsion without conjugated AMB8LK), labeled with coumarin 6, were added to 1 X 10⁶ CAPAN-1 cell and incubated for 30 min at 4°C. Cells were washed with 1 ml of immunofluorescence (IF) buffer (1% (w/v) BSA in PBS pH 7.4). Hereafter, cells that had been incubated with fluorescently labeled emulsions were re-suspended in 500 µl of IF-buffer and analyzed by flow cytometry.

### 9. Confocal laser scanning microscopy (CLSM) analysis of cellular uptake of immunoemulsions.

CAPAN-1 cells were grown on coverslips to subconfluency. Cells were incubated with coumarin 6 labeled emulsions (cationic emulsion and AMB8LK immunoemulsion) in serum-supplemented growth media at 37°C for 0 min, 30 min and 1 h, washed extensively with PBS, mounted in glycerol and observed with a confocal microscope.

### 10. Measurement of AMB8LK-immunoemulsion and drug uptake by the cells.

CAPAN-1 cells were grown to subconfluency on 24 well plates. Cells were incubated with coumarin 6 labeled emulsions (cationic emulsion, anionic emulsion and AMB8LK immunoemulsion) in PBS at 37°C for 1 h and washed extensively with PBS. Plate was taken for fluorescence measurements using Fluo Star-Galaxy from BMG Labtechnologies with excitation wavelength 485 nm and emission wavelength of 520 nm. Each plate was read 4 times and an average value was calculated. Wells, which were not washed and incubated with the same samples, served as a reference for total fluorescence.

### Example 1B: Results

### 1.1. AMB8LK Fab' production.

AMB8LK Fab' fragments production was assessed by SDS-PAGE confirming that F(ab)2 cleavage to Fab' fragments by mild reduction with mercaptoethylamine (MEA) does occur. AMB8LK Fab' antigenic specifity was verified by sandwich ELISA with ferritin as coating antigen.

Free thiol groups were determined with Aldrithiol, by monitoring the change in absorbance at 343 nm, with cysteine as standard. After this procedure the antibody exposed 3 free thiol groups.

### 1.2. AMB8LK-immunoemulsion characterization.

Different antibody densities at the o/w interface were used (from 10 up to 100 antibody molecules per oil droplet) to examine their influence on the final droplet size and zeta potential of the emulsion. It was noticed that antibody density had no effect on both droplet size and zeta potential which were 110-130 nm and +35mV respectively. The coupling efficiency, which was determined by ELISA, was also not affected by the surface antibody density. Irrespective of the initial Ab/droplet ratio the efficiency ranged from 55 to 63% as measured following unreacted Ab removal. This was further confirmed by confocal microscope and TEM observations, respectively from which it can be deduced clearly that most of the Ab molecules are attached to the oil droplets at the o/w interface while free Ab molecules are localized in the external aqueous medium.

These findings show marked improvement compared to the results obtained with intact IgG molecule. The efficiency increased from 25 to 63% while the density of the AMB8LK fragment was further increased from 40 to 100 Ab molecules/droplet. These observations point out the importance of the steric hindrance effect related to the size of the Ab molecule. Thus, the highest density was achieved with the Fab' AMB8LK fragment (MW of 50 000) as compared to IgG the MW of which was 150 000. It can be deduced that the coupling efficiency increases with the decrease in molecular weight of the MAb fragment.

### 1.3. Immunostaining of H-ferritin.

Immunostaining for the determination of the H-ferritin over-expression in various cancer cell lines such as CAPAN-1 (pancreatic cancer cells), SK-BR-3 (breast cancer cells), Caco-2 (colon cancer cells) and in two control normal cell lines such as fibroblasts and smooth muscle cells was performed. Cells were incubated with the AMB8LK Fab2 fragment in order to detect the H-Ferritin over expression. Cells which were not incubated with the AMB8LK Fab2 fragment, but only with the secondary antibody were used as a control. Clear fluorescence was noted only in the cancerous cells indicating the expression of ferritin while the normal cells did not exhibit any fluoresence confirming the absence of ferritin.

Therefore, the assumption that H-Ferritin molecule can be used as a target molecule for breast, colon and pancreas cancers therapy seems to be founded and merits further investigation.

### 1.4. In vitro binding analysis using flow cytometry.

FACS analysis revealed that the AMB8LK-immunoemulsion and the cationic emulsion bind to CAPAN-1 cell lines compared to the anionic emulsion which does not bind to the cells due to its negative charge nature.

### 1.5. In vitro uptake visualization by confocal microscope.

The AMB8LK immunoemulsion formulation qualitatively binds more rapidly and efficiently to Capan-1 cell line then the blank cationic emulsion.

### 1.6. Quantitative uptake determination.

The fraction of fluorescent lipophilic probe comprised in the emulsion which penetrates within the cells is higher with the cationic emulsion (42.7%) than with the anionic emulsion (34%) confirming previous results which already have shown that the cationic emulsion enhance the penetration of different lipophilic drugs through various tissues and organs irrespective of the route of administration as compared to the anionic emulsion [22]. It can be deduced that the coupling of AMB8LK to the cationic emulsions not only did not prevent or decrease the uptake of the fluorescent probe but on the contrary increased it significantly (by 50%).

The enhanced penetration of the probe clearly indicated that the internalization process is not only mediated by an electrostatic effect of the cationic oil droplets but probably also by a cell-receptor mediated endocytosis through a cell surface internalizing receptor ligand effected by AMB8LK MAb.

### Example 2: Conjugation method using N-(1-stearyl)-maleimide.

### 2.1. Synthesis of cross-linker N-(1-stearyl)-maleimide (SM) .

A mixture of stearylamine (0.01 mole) and maleic anhydride (0.01 mole) in chloroform (10 ml) was stirred over 3 h at room temperature. The deposited crystals were filtered off and washed with small amount of chloroform to give the pure N-(1-stearyl) maleimic acid. The latter compound (4 mmole) and sodium acetate (0.03 g, equivalent to 0.3 mmole) were refluxed in acetic anhydride (30 ml) over 30 min and then immediately cooled on an ice bath. The deposited crystals were collected and washed with water to give the title compound N-(1-stearyl)-maleimide with a yield of 85% (scheme-1).

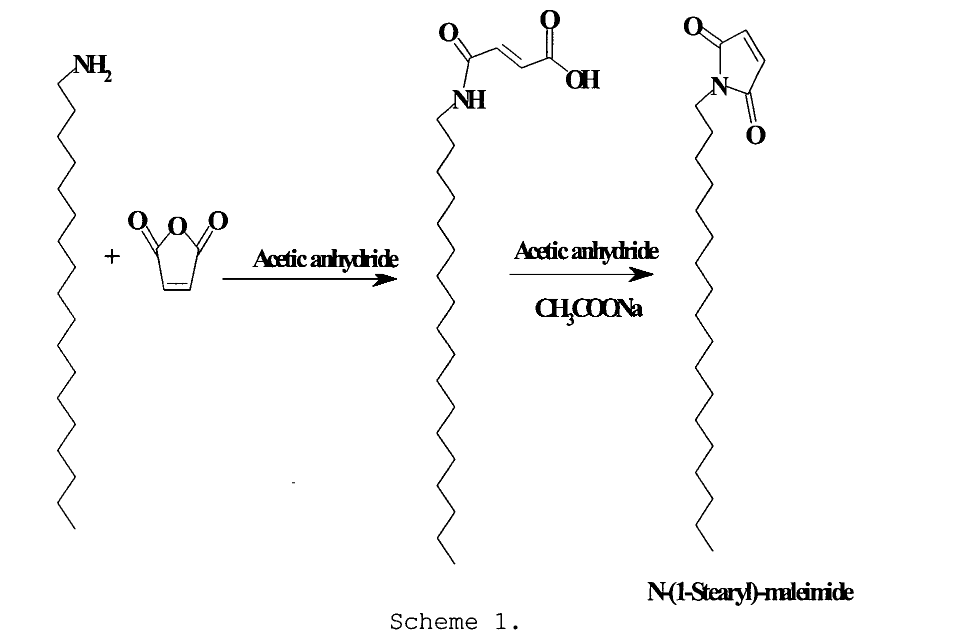

### 2.2. Conjugation of trastuzumab antibody to the emulsion via thioether method.

### 2.2.1. Thiolation of trastuzumab.

The antibody was thiolated using 2-iminothiolane (Traut's reagent, Aldrich Chemicals co. Milwaukee Wisconsin). The antibody was dissolved in buffer solution pH 8.5 composed of 50 mM Tris, 1 mM EDTA and 150 mM NaCl. Then 2-iminothiolane was added in a molecular ratio of 600:1 with respect to the antibody followed by incubation over 45 min at room temperature. The reaction mixture was applied to HiTrap™ Desalting column (Amersham Bioscience, Uppsala, Sweden) to remove excess of 2-iminothiolane.

The modification of the antibody by Traut's reagent did not affect its ability to recognize specifically the antigen HER2 over-expressed on SK-BR3 cells. There is no fluorescence in the absence of trastuzumab whereas in the presence of trastuzumab, a marked cell surface fluorescence is noted.

### 2.2.2. Conjugation method.

The thiolated antibody was immediately added to the cationic emulsion (pH 6.5) containing N-(1-stearyl)-maleimide (SM) cross-linker. The reaction mixture was left overnight at room temperature while mixing and under nitrogen atmosphere. The unconjugated antibody was removed by eluting the mixture through a 1.5X25 cm Sepharose CL-4B column (Amersham Bioscience, Uppsala, Sweden) with water.

### 2.3. Evaluation of the conjugation reaction efficacy.

### 2.3.1. ELISA.

The conjugation efficacy (the percent of conjugation of the antibody to the emulsion) was evaluated as a function of the concentration of the new cross-linker N-(1-stearyl)-maleimide (SM) using the ELISA (Enzyme-linked Immunosorbent Assay) which is a semi-quantitative method according to the following protocol:
- Coating the wells with the immuno-emulsions or blank emulsion (diluted in coating buffer pH 9.5),
- Incubation overnight at 37°C,
- Washing with PBS-Tween 20 X 3,
- Blocking non-specific binding by BSA 2% over 1.5 h,
- Washing with PBS-Tween 20 X 3,
- Adding secondary antibody (Horseraddish conjugated anti-human IgG, Jackson Immunoresearch Ltd., West Grove, Pennsylvania, USA) and incubation for 2 hr RT,
- Washing with PBS-Tween20 X 3,
- Adding substrate (TMB/E, Single Oak Drive - Temecula, California),
- Reading UV absorbance of the developing color at 650 nm using spectrofluorometer.

The conjugation efficacy increased with increasing amounts of SM cross-linker in the emulsion preparation reaching almost 30% conjugation with an initial ratio of 45 trastuzumab molecules per oil droplet.

### 2.3.2. Immuno-gold staining.

A 12 nm gold-conjugated secondary anti-human IgG antibody (Jackson Immunoresearch Ltd., West Grove, Pennsylvania, USA) was used to detect trastuzumab on the oil droplets of the emulsion using the technique previously reported by other authors (6). Following an immunoreaction between trastuzumab and the gold secondary antibody, black dots which are in fact gold nanoparticles can be visualized and localized using Transmission Electron Microscopy.

It can be clearly observed that trastuzumab is located on the oil droplets of the emulsion. Furthermore, the number of black dots on a single oil droplet is close to up to 5-6 Ab molecules per droplet. In addition, there are few free black dots in the external aqueous phase of the emulsion and a random conjugation occurred. These results confirm the ELISA findings.

### Example 3: Measure of activity.

### 3.1. Cell culture studies.

The well established cell line model for breast cancer cells, SK-BR3 cells (ATCC, Manassas, Virginia, USA) which are known to over-express HER2 antigen, were used throughout the entire study to evaluate the trastuzumab affinity and specific activity following of the immunoemulsion preparation. 3.2. Binding studies.

The cells were fixed on cover-slips, non-specific binding was blocked with 5% BSA. Then, the cells were incubated overnight with either blank emulsion or immuno-emulsion (final dilution 1:1000). Three successive washings were performed with PBS to remove unbound oil droplets and then FITC-conjugated anti-human antibody (1:50 final dilution) was added to detect trastuzumab. An additional washing was done.

Fluorescence was visualized using Zeiss Confocal Microscope. The antibody is attached to the emulsion and bound to cells. Furthermore, since the secondary antibody recognized trastuzumab, this indicates that the trastuzumab did not denaturate following the preparation procedures.

However, in order to visualize the blank emulsion also, the emulsions were labeled with lipophilic fluorescent probe coumarin-6 (Polyscience Inc, Warrington, Pennsylvania) in a ratio of 1:1000 coumarin to Lipoid E80 in the oil phase (7).

Cells were fixed on cover-slips, non-specific binding was blocked with 5% BSA. Incubation over 1 h with the labeled blank emulsion and immuno-emulsion (final dilution of 1:1000) was carried out at room temperature.

The cells were washed three times successively with PBS and then observed using Confocal microscope. A qualitative significant difference in binding to SK-BR3 cells between blank emulsion and the immuno-emulsion is observed as reflected by the difference in the intensity of the fluorescence. It can be clearly seen that the immuno-emulsion binds extensively to the cells, while the blank emulsion binding is minimal over 1 hour incubation at room temperature.

For confirmation purposes, additional studies were performed using FACS analysis (Fluorescent activated cell sorting analysis). Cells were fixed, incubated with either trastuzumab alone or with the immuno-emulsion (with the same concentration of trastuzumab in both samples) over 1 hour. The cells were washed with PBS three times successively and then FITC-conjugated secondary antibody was added (in a final dilution of 1:50). Cells were washed again with PBS and then were analyzed by FACS.

Apparently the affinity extent of the immunoemulsion as compared to the same quantity of the free antibodies ranged around 5%.

Nevertheless, a significant binding of the oil droplets as compared to control occurred and may promote the internalization of marked drug amount loaded in the cationic oil droplets.

### 3.3. Uptake studies.

SK-BR3 cells were incubated at 37°C with either blank emulsion or immuno-emulsion labeled with coumarin-6, over different incubation times: 5, 15, 30 min.

Cells were washes with PBS three times successively and then were fixed and visualized with Zeiss Confocal Microscope.

It can be clearly noted that the blank emulsion binds to cells for some minimal extent while qualitatively the immuno-emulsion is bound more strongly to the cells and binding extent is more pronounced with time.

### Example 4: Conjugation method using SMCC.

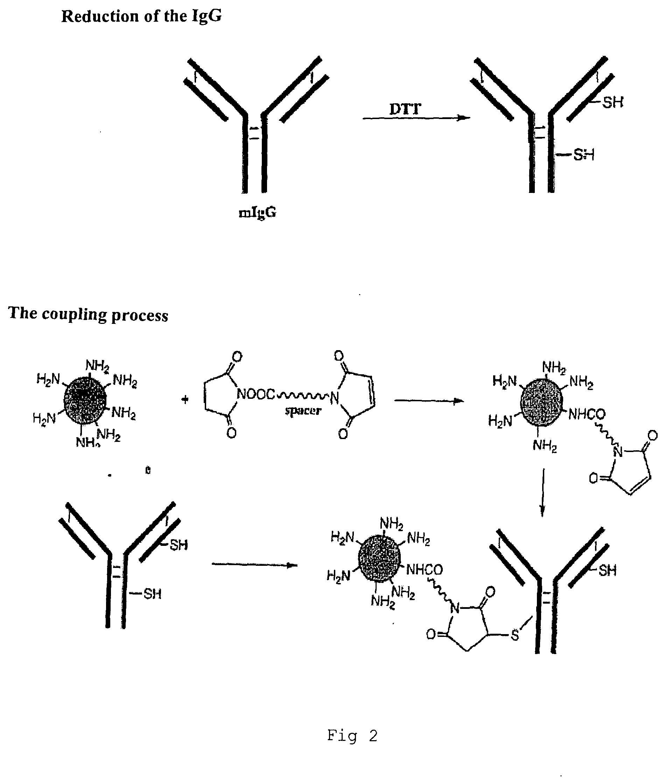

It is illustrated in figure 2. The coupling of the SMCC to stearylamine (SA) can be done using two different approaches. The emulsion is first prepared and the SMCC is added to the emulsion, so the reaction will occur on the o/w interface. Alternatively SMCC can be first attached to native SA and the novel coupling agent is being incorporated in the oil phase prior to the emulsion formation. In a second phase the antibody is being reduced under mild conditions in the presence of 1,4-Dithioerythritol (DTT).

A 2 mg/ml solution of antibody in PBS buffer was incubated with 20 mM DTT for 30 min at room temperature or 50 nM mercaptoethylamine for 1 hour at 37°C with stirring. The solution of partially reduced antibody was then gel filtrated using Sephadex G25, in order to remove excess of unreacted of DTT and to exchange the solution medium with 2.25% glycerol solution (PBS free). 100 g of the reduced antibody was then added to 10 ml of maleimide derivatized emulsion (activated emulsion) under constant stirring overnight at room temperature.

### Example 5: Conjugation method using SPDP.

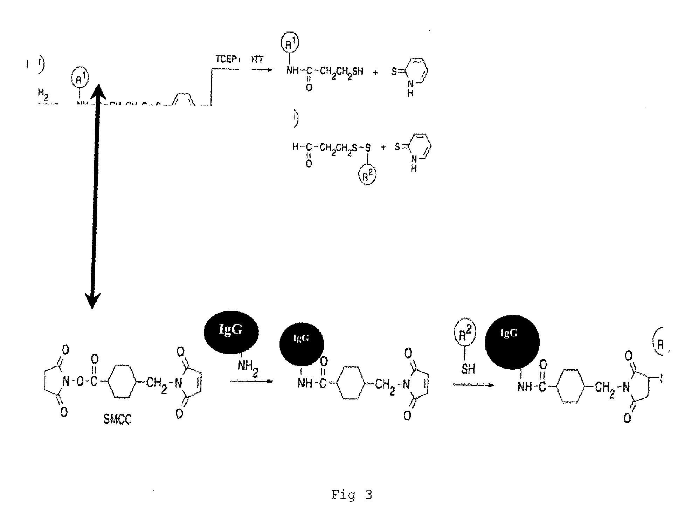

It is illustrated in figure 3. SPDP will first be added to the emulsion to react with the NH₂ moieties of SA at the o/w interface resulting in the formation of disulfide bonds. The mixed disulfide bond will then react with reducing agent (DTT) over 2 hours, resulting in a release of pyridyldithiopropionyl conjugate and formation of free SH group on droplet s surface. At the end of this procedure a vivaspin20 (10kDa) dialysis will be preformed in order to remove the pyridyldithiopropionyl by product, unreacted DTT and any possible excess of SPDP. The emulsion (with free SH groups) will then be incubated with activated antibody (IgG reacted with SMCC) to yield the final antibody-emulsion conjugation.

### Example 6: Conjugation of AMB8LK.

### 6.1.1. Emulsion preparation.

AMB8LK immunoemulsion was prepared as previously described. Fluorescent emulsions were prepared using Coumarin 6 probe. The probe was incorporated into the emulsions at a molar ratio of 1:200. Since the MCT concentration was 5% (~97.6µmol/ml), the amount of Coumarin 6 added, was 0.488µmol/ml. Briefly, 4 µl of a 9.3 mM solution of Coumarin 6 in tetrahydrofuran (THF) were added to 500ul of emulsion after Sepharose CL-4B separation. The mixture was vortexed and incubated at 37°C for 2 hours.

### 6.1.2. FACS analysis.

For FACS analysis Capan-1 cell line were grown to reach confluence. Cells were trypsinized and centrifuged at 1 200 rpm for 5 min. The supernatant was removed and 1 ml of FACS buffer was added. All the time, cells were kept on ice. 1-2X10⁵ cells in FACS tubes were washed and centrifuged again, then taken per stain in 100 µl of FACS buffer containing different emulsion formulations in a dilution of 1:1 000. The incubation time was for 30 min on ice. Cells were washed again, centrifuged and resuspended in 0.5-1 ml of FACS buffer. Afterwards the FACS analysis was preformed.

### 6.1.3. Fluorescent microscopy analysis.

Capan-1 cells were trypsinized after reaching confluence and were transferred (10⁵ cells per well) into 16 wells plate, which was covered with 18mm cover slips.

Cells were left to adhere to the cover slip for 24 hours, 37°C, and 5% CO₂. After 24 hours, medium was discarded and cells were incubated with labeled emulsion 1:1000 in PBS for 0, 30 and 60 min at 37°C. Later, fixation was done using fresh 4% paraformaldehyde for 10 min. Paraformaldehyde was discarded and cells were washed with PBS X3. Self-fluorescence was blocked with 50mM NH₄Cl in PBS for 5 min. Cells were washed again in PBSX3 and were taken for observation with fluorescence microscope and confocal microscope.

### 6.1.4. Quantitative analysis.

Capan-1 cells were trypsinized after reaching confluence and were transferred (10⁵ cells per well) into 24 wells plate. Cells were left to adhere to the plate up to reaching confluency at 37°C, and 5% CO₂. In the next step, the various labeled emulsion samples were incubated with cells (1:1000 in PBS) over 45 min at 37°C. Cell samples were washed in PBSX3 while the controls were not washed. Plate was taken for fluorescence measurements using Fluo Star- Galaxy from BMG Lab technologies with excitation wavelength 485 nm and emission wavelength of 520nm. Each plate was read 4 times and an average value was calculated.

### 6.2. Results.

### 6.2.1. Labeling of emulsions.

Confocal microscopy observations lead to conclude that oil droplets are labeled with Coumarin 6.

### 6.2.2. Comparison between AMB8LK immunoemulsion and a cationic emulsion (labeled formulations).

They were incubated with Capan-1 cells for different time intervals 0, 30 and 60 min at 37°C. The comparison between two formulations was carried out using confocal and fluorescent microscopes.

### 6.2.3. Binding studies of cationic emulsions, anionic emulsions and immunoemulsions.

The binding of different emulsion preparations was also studied using FACS analysis (4°C, 30 min). All the formulations, except the anionic one bind to Capan-1 cell line at 4°C for 30 min. The difference between the cationic emulsion and AMB8LK immunoemulsion was better observed when the incubation took place in 37°C for 1 hour.

### 6.2.4. Uptake studies.

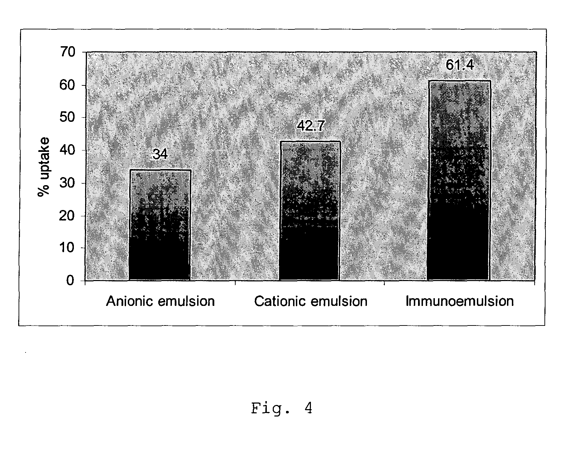

For quantitative uptake determination of emulsion samples by Capan-1 cells fluorescence measurements was done using FlouStar-Galaxy. It can be noted from the data presented in figure 4 that the fraction of fluorescent lipophilic probe comprised in the emulsions which penetrates within the cells is higher with the cationic emulsions than with the anionic emulsion. The coupling of AMB8LK to the cationic emulsions, not only did not prevent or decrease the uptake of the fluorescent probe, but on the contrary enhanced significantly (by 50%) the penetration of the probe clearly indicating that the internalization process is not only mediated by an electrostatic effect of the cationic oil droplets but also by a cell receptor-mediated endocytosis through a cell surface internalizing receptor ligand effected by AMB8LK monoclonal antibody.

### REFERENCES

1. Allen TM. Ligand-targeted therapeutics in anticancer therapy. Nat Rev Cancer. 2002 Oct; 2(10):750-63.
2. Farah RA, Clinchy B, Herrera L, Vitetta ES. The development of monoclonal antibodies for the therapy of cancer, Crit Rev Eukaryot Gene Expr. 1998;8(3-4):321-56.
3. Olayioye MA. Update on HER-2 as a target for cancer therapy: Intracellular signaling pathways of ErbB2/HER-2 and family members. Breast Cancer Res. 2001;3(6):385-9. Epub 2001 Oct 04.
4. Slamon DJ, Clark GM, Wong SG, Levin WJ, Ullrich A, McGuire WL. Human breast cancer: correlation of relapse and survival with amplification of the HER-2/neu oncogene. Science, 1987 Jan 9;235(4785):177-82.
5. Slamon DJ, Godolphin W, Jones LA, Holt JA, Wong SG, Keith DE, Levin WJ, Stuart SG, Udove J, Ullrich A, et al. Studies of the HER-2/neu proto-oncogene in human breast and ovarian cancer. Science. 1989 May 12;244(4905):707-12.
6. Ross JS, Gray K, Gray GS, Worland PJ, Rolfe M. Anticancer antibodies, Am J Clin Pathol. 2003 Apr;119(4):472-85.
7. Merlin JL, Barberi-Heyob M, Bachmann N, In vitro comparative evaluation of trastuzumab (Herceptin) combined with paclitaxel (Taxol) or docetaxel (Taxotere) in HER2-expressing human breast cancer cell lines. Ann Oncol. 2002 Nov;13(11):1743-8.
8. Nishimura R, Nagao K, Miyayama H, Okazaki S, Nakagawa K, Fujimura Y. A case of recurrent breast cancer with lung metastasis and overexpression of HER2 that responded to UFT and cyclophosphamide combination therapy after sequential treatments with epirubicin, taxanes, and trastuzumab, Gan To Kagaku Ryoho. 2003 Jun;30(6):879-82.
9. Kadouche J, Levy R. Utilisation d'anticorps monoclonaux anti-ferritiens dans le traitement de certains cancers. 2002. FR 00 00718.
10. Vriesendorp HM, Quadri SM. Radiolabeled immunoglobulin therapy: old barriers and new opportunities. Expert Rev Anticancer Ther. 2001 Oct;1(3):461-78.
11. Alan R, et al. Distribution of monoclonal antiferritin antibody in Kaposi's sarcoma, Hodjkin's disease and hepatocellular carcinoma. Human Pathology. 2003 April;34:381-84.
12. Park JW, Kirpotin DB, Hong K, Shalaby R, Shao Y, Nielsen UB, Marks JD, Papahadjopoulos D, Benz CC. Tumor targeting using anti-her2 immunoliposomes. J Control Release. 2001 Jul 6;74(1-3):95-113.
13. Park JW, Hong K, Kirpotin DB, Colbern G, Shalaby R, Baselga J, Shao Y, Nielsen UB, Marks JD, Moore D, Papahadjopoulos D, Benz CC. Anti-HER2 immunoliposomes: enhanced efficacy attributable to targeted delivery. Clin Cancer Res. 2002 Apr;8(4):1172-81.
14. Nam SM, Kim HS, Ahn WS, Park YS. Sterically stabilized anti-G(M3), anti-Le(x) immunoliposomes: targeting to B16BL6, HRT-18 cancer cells. Oncol Res. 1999;11(1):9-16.
15. Maruyama K, Takahashi N, Tagawa T, Nagaike K, Iwatsuru M. Immunoliposomes bearing polyethyleneglycol-coupled Fab' fragment show prolonged circulation time and high extravasation into targeted solid tumors in vivo. FEBS Lett. 1997 Aug 11;413(1):177-80.
16. Koning GA, Morselt HW, Velinova MJ, Donga J, Gorter A, Allen TM, Zalipsky S, Kamps JA, Scherphof GL. Selective transfer of a lipophilic prodrug of 5-fluorodeoxyuridine from immunoliposomes to colon cancer cells. Biochim Biophys Acta. 1999 Aug 20;1420(1-2):153-67.
17. Lopes de Menezes DE, Pilarski LM, Allen TM. In vitro and in vivo targeting of immunoliposomal doxorubicin to human B-cell lymphoma, Cancer Res. 1998 Aug 1;58(15):3320-30.
18. Mastrobattista E, Storm G, van Bloois L, Reszka R, Bloemen PG, Crommelin DJ, Henricks PA. Cellular uptake of liposomes targeted to intercellular adhesion molecule-1 (ICAM-1) on bronchial epithelial cells. Biochim Biophys Acta. 1999 Jul 15;1419(2):353-63.
19. Mastrobattista E, Koning GA, Storm G. Immunoliposomes for the targeted delivery of antitumor drugs. Adv Drug Deliv Rev. 1999 Nov 10;40(1-2):103-127.
20. Constantinides PP, Lambert KJ, Tustian AK, Schneider B, Lalji S, Ma W, Wentzel B, Kessler D, Worah D, Quay SC. Formulation development and antitumor activity of a filter-sterilizable emulsion of paclitaxel. Pharm Res. 2000 Feb;17(2):175-82.
21. Lundberg BB, Griffiths G, Hansen HJ. Conjugation of an anti-B-cell lymphoma monoclonal antibody, LL2, to long-circulating drug-carrier lipid emulsions. J Pharm Pharmacol. 1999 Oct;51(10):1099-105.
22. Shi Cheng Yang, Simon Benita. Enhanced absorption and drug targeting by positively charged submicron emulsions, Drug Dev. Res. 2000. 50:476-486.
23. Benita S. Prevention of topical and ocular oxidative stress by positively charged submicron emulsion. Biomed Pharmacother. 1999 May;53(4):193-206.
24. Ezra R, Benita S, Ginsburg I, Kohen R. Prevention of oxidative damage in fibroblast cell cultures and rat skin by positively-charged submicron emulsion of α-tocopherol. Eur. J. Pharm. Biopharm. 1996;42(4):291-298.
25. Hewitt RE, Powe DG, Carter GI, Turner DR, Price JE. Basement membrane collagen-IV synthesis in colorectal tumours. Int J Cancer. 1992 Jun 19;51(4):530-6.
26. Hazard JT, Drysdale JW. Ferritinaemia in cancer. Nature, 1977 Feb 24;265(5596):755-6.
27. Vaughn CB, Weinstein R, Bond B, Rice R, Vaughn RW, McKendrick A, Ayad G, Rockwell MA, Rocchio R. Ferritin content in human cancerous and noncancerous colonic tissue. Cancer Invest. 1987;5(1):7-10.
28. Cohen C, Shulman G, Budgeon LR. Immunohistochemical ferritin in testicular seminoma. Cancer. 1984 Nov 15;54(10):2190-4.
29. Guner G, Kirkali G, Yenisey C, Tore IR. Cytosol and serum ferritin in breast carcinoma. Cancer Lett. 1992 Dec 24;67(2-3):103-12.
30. Weinstein RE, Bond BH, Silberberg BK, Vaughn CB, Subbaiah P, Pieper DR. Tissue ferritin concentration and prognosis in carcinoma of the breast. Breast Cancer Res Treat. 1989 Dec;14(3):349-53.
31. Lai J, Quadri SM, Borchardt PE, Harris L, Wucher R, Askew E, Schweichel L, Vriesendorp HM. Pharmacokinetics of radiolabeled polyclonal antiferritin in patients with Hodgkin's disease. Clin Cancer Res. 1999 Oct;5(10 Suppl):3315s-3323s.
32. Vriesendorp HM, Quadri SM, Andersson BS, Wyllie CT, Dicke KA. Recurrence of Hodgkin's disease after indium-111 and yttrium-90 labeled antiferritin administration. Cancer. 1997 Dec 15;80(12 Suppl):2721-7.
33. Vriesendorp HM, Quadri SM, Stinson RL, Onyekwere OC, Shao Y, Klein JL, Leichner PK, Williams JR. Selection of reagents for human radioimmunotherapy. Int J Radiat Oncol Biol Phys. 1992;22(1):37-45.
34. Vriesendorp HM, Quadri SM, Wyllie CT, Lai J, Borchardt PE, Harris L, Wucher R, Askew E, Schweichler L. Fractionated radiolabeled antiferritin therapy for patients with recurrent Hodgkin's disease. Clin Cancer Res. 1999 Oct;5(10 Suppl):3324s-3329s.
35. Chan CC, Rozenszajn LA, Nussenblatt RB, Muellenberg-Coulombre C, Hsu SM, Palestine AG, Lando Z, BenEzra D. Monoclonal antibodies to Muller's cells of the retina.Invest Ophthalmol. Vis. Sci., 1984 Sep; 25(9): 1007-12.
36. Greg T Hermanson. Bioconjugate Techniques. Academic Press, 1996, 463-464.
37. Levy M.Y, Schutze W, Fuhrer C and Benita S, Characterization of diazepam submicron emulsion interface, the role of oleic acid, J. Microencapsulation, 1994, 11: 79-92.
38. Andreeva M, Niedmann PD, Binder L, Armstrong VW, Meden H, Binder M, Oellerich M. A simple and reliable reverse-phase high-performance liquid chromatographic procedure for determination of paclitaxel (taxol) in human serum. Ther Drug Monit. 1997 Jun;19(3):327-32.

## Claims

1. A combination product comprising a positive oil in water emulsion wherein said emulsion comprises a compound presenting free NH₂ groups, at its natural state, at the oil-water interface, and an antibody, wherein said compound is linked to said antibody by a heterobifunctional linker, linking said NH₂ groups to SH groups on the antibody hinge region.

2. The combination product of claim 1 wherein said product has a positive zeta charge.

3. The combination product of claim 1 or 2, wherein said compound presenting NH₂ free groups is at least one cationic lipid selected from the group consisting of a C₁₀-C₂₄ alkylamine, a C₁₀-C₂₄ alkanolamine and a cholesterol ester.

4. The combination product of claim 3, wherein said compound presenting NH₂ free groups is stearylamine or oleylamine.

5. The combination product of any of claims 1 to 4, wherein said emulsion comprises colloid particles having an oily core surrounded by an interfacial film, wherein said interfacial film comprises said compound presenting free NH₂ at its natural state, nonionic surfactant and an anionic surfactant or anionic lipid, wherein said colloidal particles have a positive zeta potential.

6. The combination product of 5, wherein said emulsion contains an active principle (drug).

7. The combination product of any of claims 1 to 6, wherein said antibody is a polyclonal antibody.

8. The combination product of any of claims 1 to 6, wherein said antibody is a monoclonal antibody selected from the group comprising native forms, synthetic forms, chimeric forms and humanized forms.

9. The combination product of any of claims 1 to 8, wherein said antibody targets an antigen present at the surface of a pathological cell.

10. The combination product of any of claims 1 to 9, wherein said antibody targets a protein selected from the group comprising HER-2, H-ferritin, PSMA, mucins, MUC 1, CD 44 and retinal S-Ag.

11. The combination product of any of claims 1 to 6 and 8 to 10, wherein said antibody is AMB8LK antibody.

12. The combination product of any of claims 1 to 11, wherein said linker is chosen from N-1 stearyl-maleimide (SM), oleylmaleimide, succunimidyl trans-4-(maleimidylmethyl)cyclohexane-1-carboxylate (SMCC) and succinimidyl 3-(2-pyridyldithio)propionate (SPDP).

13. A method for producing a combination product according to claim 1, comprising the steps of:
a) optionally reducing an antibody in order to obtain free SH group on its hinge region,
b) mixing a positive emulsion wherein said emulsion comprises a compound which, at its natural state, contains free NH₂ groups, wherein said compound is linked to a heterobifunctional linker by said NH₂ groups, with the antibody presenting free SH groups in order to obtain said combination product.

14. The method of claim 13, wherein said positive emulsion in step b) is obtained by:
i. linking an linker to a free NH₂ group naturally present on a compound that is used to obtained a positive emulsion, in order to obtain a modified compound,
ii. mixing said modified compound, which at its natural state contains free NH₂ groups, with the other products necessary to obtain an emulsion, in order to obtain a positive emulsion.

15. The method of claim 13, wherein said positive emulsion in step b) is obtained by:
i. mixing a compound, which at its natural state contains free NH₂ groups, with the other products necessary to obtain an emulsion, in order to obtain a positive emulsion,
ii. linking a linker to a free NH₂ group naturally present on said compound, in order to obtain a modified compound within said positive emulsion.
